# EUROPÄISCHE PATENTSCHRIFT

(11) **EP 0 759 169 B1**
(45) Veröffentlichungstag und Bekanntmachung des Hinweises auf die Patenterteilung: **14.01.1998**
(21) Anmeldenummer: 95918521.6
(22) Anmeldetag: 05.05.1995
(51) Int. Cl.: G01N 33/497, G01N 1/42

(54) **VERFAHREN UND VORRICHTUNG ZUM SAMMELN VON AUSGEATMETEM ATEMKONDENSAT**
PROCESS AND DEVICE FOR COLLECTING EXPIRED BREATH CONDENSATE
PROCEDE ET DISPOSITIF PERMETTANT DE RECUEILLIR UN CONDENSAT DE SOUFFLE EXPIRE

(30) Priorität: 13.05.1994 DE 4417282; 13.05.1994 DE 9421065 U; 10.02.1995 DE 19505504
(43) Veröffentlichungstag der Anmeldung: 26.02.1997
(73) Patentinhaber: FILT FORSCHUNGSGESELLSCHAFT FÜR LUNGEN- UND THORAXERKRANKUNGEN MBH, 13122 Berlin (DE)
(72) Erfinder: WINSEL, Klaus, D-10243 Berlin (DE); BECHER, Gunther, D-16321 Schönow (DE); STRESEMANN, Ernst, D-32105 Bad Salzuflen (DE); NEUBAUER, Günter, D-13125 Berlin (DE)
(74) Vertreter: Röhnicke, Heinz
(86) Internationale Anmeldenummer: DE9500633
(87) Internationale Veröffentlichungsnummer: WO9531721

(56) Entgegenhaltungen:
- EP-A- 0 275 829
- WO-A-91/05255

## Beschreibung

Die Erfindung bezieht sich auf ein Verfahren und eine Vorrichtung zum Sammeln von ausgeatmetem Atemkondensat für die Diagnose des Gesundheitszustandes und von Stoffwechselleistungen der Lunge und der Atemwege sowie anderer Organe und zur Analyse ausgeatmeter körperfremder Stoffe, wobei die Ausatemluft gekühlt und die nicht gasförmigen Bestandteile in einem Probengefäß auskondensiert werden.

Durch die WO 91/05255 Al ist ein Verfahren zur Diagnose des Gesundheitszustandes sowie zur Therapieüberwachung und zur Verlaufskontrolle von Krankheiten, speziell der Lunge und der Atemwege, bekannt, bei dem nicht-flüchtige Substanzen - gegebenenfalls zusammen mit endogenen und exogen flüchtigen Stoffen - in der menschlichen Atemluft analysiert werden. Hierzu werden von einem Probanden AtemstÖße auf ein auf -196 °C gekühltes, 1 cm² großes Probenträgerplättchen aufgehaucht. Die Probe wird dann auf dem Cryotisch durch Anlegen eines Ultrahochvakuums gefriergetrocknet und anschließend in unterschiedlicher Weise analysiert.

Die Herstellung einer Probe bei einer Temperatur von -196 °C beschränkt das Verfahren auf mit speziellen, kostenaufwendigen Vorrichtungen ausgestattete medizinische Einrichtungen.

Es ist weiterhin ein Verfahren zum Sammeln von ausgeatmetem Atemkondensat bekannt (Am. Rev. Repir. Dis. Vol. 148, 1993, S. 956). Hierbei wird durch eine zu untersuchende Person über ein Mundstück durch ein Rohr aus Polyvinylchlorid ausgeatmet. Dieses Rohr ist mit Eis gekühlt, so daß sich ein Atemkondensat bildet, welches in eine eisgekühlte Flasche tropft und in dieser sich sammelt. Für die Analyse ist eine Aufbereitung der Probe erforderlich. Die Abkühlungstemperatur der Ausatemluft liegt dabei über 0 °C.

Die Aufbewahrung einer derartigen Probe ist nur kurzzeitig möglich. Sie erfordert eine relativ aufwendige Aufbereitung. Weiterhin verbleiben in dem Rohr größere Mengen des Atemkondensates.

Der Erfindung liegt die Aufgabe zugrunde, ein Verfahren und eine Vorrichtung zum Sammeln von ausgeatmetem Atemkondensat für die Diagnose des Gesundheitszustandes und von Stoffwechselleistungen der Lunge und der Atemwege sowie anderer Organe und zur Analyse ausgeatmeter körperfremder Stoffe, wobei die Ausatemluft gekühlt und die nicht gasförmigen Bestandteile in einem Probengefäß auskondensiert werden, zu schaffen, wobei das Atemkondensat weitgehend vollständig und für längere Zeit ohne Nachbehandlung aufbewahrbar ist und dabei die Vorrichtung einen einfachen Aufbau aufweist, wodurch sie auch in kleineren medizinischen Einrichtungen einsetzbar ist.

Erfindungsgemäß wird die Aufgabe dadurch gelöst, daß die Ausatemluft ein Probensammelrohr durchströmt und in diesem auf eine Minustemperatur unter 0 °C abgekühlt wird, wobei die flüssigen und lösbaren Bestandteile auskondensieren und an der, eine niedrigere Temperatur als das Atemkondensat aufweisenden, Innenwand des Probensammelrohres anfrieren. Vorzugsweise wird die Ausatemluft in dem Probensammelrohr auf eine Temperatur von bis -5 °C abgekühlt, und die Temperatur seiner Innenwand beträgt zwischen -10 °C und -25 °C.

Bei dieser Temperatur sind die Bildung des Atemkondensates und ein Anfrieren an die Innenwandung des Probensammelrohres gewährleistet. Dabei ist es möglich, daß entlang dem Probensammelrohr an der Kondensierstrecke der Ausatemluft unterschiedliche Temperaturgradienten eingestellt werden.

Besonders vorteilhaft ist es, wenn das Probensammelrohr während der Auskondensierung kontinuierlich gekühlt wird.

Um in ausreichendem Maße Atemkondensat für Analysen zur Verfügung zu haben, sollte in dem Probensammelrohr in einem Zeitraum von bis zu 15 min bis zu fünfmal Primärkondensat gesammelt werden.

Um das gesamte Atemkondensat aufzufangen, kann dem Probensammelrohr ein Sammelgefäß nachgeschaltet werden, in dem sich bildendes Atemkondensat aufgefangen wird. Hierbei handelt es sich um geringe Mengen, die beispielsweise zu Beginn des Sammelns auftreten können oder wenn die vorbestimmte Sammelmenge überschritten wird.

Zweckmäßig ist es, wenn das Probensammelrohr mit dem Atemkondensat in gefrorenem Zustand sowie mögliche Reste des Atemkondensates in dem Sammelgefäß verschlossen und in einem Tiefkühlschrank bis zu einer Analyse bzw. zur weiteren Aufbereitung für eine Analyse aufbewahrt werden. Die Aufbewahrungszeit kann dabei bis zu drei Jahren betragen.

Eine weitere Möglichkeit besteht darin, daß das Probensammelrohr aus mehreren unabhängigen Teilen gebildet wird, in denen unter gleichen Bedingungen zur gleichen Zeit das Atemkondensat gesammelt und getrennt aufbewahrt wird.

In weiterer Ausbildung der Erfindung ist eine Vorrichtung zur Durchführung des Verfahrens vorgesehen, wobei das Probensammelrohr in einem sich entlang seiner Längsachse erstreckenden Kühlelement herausnehmbar angeordnet ist, wobei das Kühlelement mit einem Elektrik- und Steuerteil verbunden ist, und dabei an dem vorderen Ende der Vorrichtung ein Mundstück sowie ein Einlaßventil zur Zuführung von Frischluft in die Einatemluft und am hinteren Ende derselben ein Auslaßventil für die gasförmigen Bestandteile der Ausatemluft angeordnet sind.

Die erfindungsgemäße Vorrichtung weist einen relativ einfachen Aufbau auf und ermöglicht es, das Atemkondensat durch Anfrieren an die Innenwand des Probensammelrohres zuverlässig zu sammeln. Über das Mundstück ist dabei eine einfache und unbehinderte Beatmung möglich.

Vorteilhafterweise besteht das Probensammelrohr aus einem biologisch inerten, kälte- und gegen Chemikalien beständigen Kunststoff, an dessen Oberfläche ein guten Anfrieren und nach dem Auftauvorgang ein vollständiges Lösen des Atemkondensates möglich ist. Vorzugsweise ist der Kunststoff ein Polytetrafluoräthylen.

Um allen Anforderungen zu entsprechen, kann die Länge des Probensammelrohres zwischen 10 und 100 cm und sein Innendurchmesser bis zu 2 cm betragen.

Eine bevorzugte Ausbildung der Vorrichtung besteht darin, daß das Kühlelement als ein Kühlmantelrohr ausgebildet ist, in dessen vorderem und hinterem Bereich jeweils eine Leitung angeordnet ist, über die es mit einem Kühlaggregat verbunden und von einer das Probensammelrohr umfließenden Kühlflüssigkeit durchströmt ist. Dabei ist der Elektrik- und Steuerteil vorzugsweise an das Kühlaggregat angeschlossen und regelt über diesen die Kühlung der Ausatemluft durch das Kühlelement.

Für eine optimale Kühlleistung ist es zweckmäßig, wenn die Ausatemluft im Probensammelrohr und die Kühlflüssigkeit im Innenraum des Kühlmantelrohres im Gegenstromprinzip zueinander fließen.

Dabei ist es möglich, daß in kostengünstiger Weise die Kühlflüssigkeit aus destilliertem Wasser mit einem Zusatz von Frostschutzmitteln gebildet ist, wobei der Gefrierpunkt derselben unter -25 °C liegt.

Eine weitere bevorzugte Ausbildung der Vorrichtung besteht darin, daß das, das Probensammelrohr umschließende, Kühlelement als ein piezoelektrischer Kälteteil ausgebildet ist, in dem ein oder mehrere piezoelektrische Elemente zur Kühlung angeordnet sind.

Hierbei können die piezoelektrischen Elemente zur Kühlung unterschiedlich regelbar sein und über diese der piezoelektrische Kälteteil entlang dem Probensammelrohr unterschiedliche Temperaturgradienten aufweisen.

Es ist auch möglich, daß das, das Probensammelrohr umschließende, Kühlelement als ein Peltier-Element für Kühlung ausgebildet ist. Dabei sind der piezoelektrische Kälteteil sowie das Peltier-Element für Kühlung über ein Leitungssystem mit dem Elektrik- und Steuerteil verbunden. Es sind in an sich bekannter Weise eine elektrische Zuführung und eine Wärmeableitung vorgesehen.

Zur vollständigen Erfassung des Atemkondensates kann am hinteren Ende der Vorrichtung unterhalb des hinteren Endes des Probensammelrohres ein abnehmbares Sammelgefäß zur Aufnahme von möglichen Resten des Atemkondensates angeordnet sein, wobei dieses aus dem gleichen Material wie das Probensammelrohr besteht. Vorzugsweise sind das Einlaßventil und das Auslaßventil als Rückschlagventile ausgebildet.

Zur Erhöhung der Wirksamkeit des Probensammelrohres ist es möglich, daß die Innenwand des Probensammelrohres als die innere Umfangsfläche desselben sowie in diesem angeordnete Zwischenwände ausgebildet ist. Damit vergrößert sich im Querschnitt die Fläche zum Anfrieren des Atemkondensates.

Um mehrere Analysen bei unter gleichen Bedingungen erfaßten Proben durchführen zu können, ist es vorteilhaft, wenn das Probensammelrohr aus mehreren unabhängigen Teilen ausgebildet ist, welche gemeinsam von dem Kühlmantelrohr umschlossen sind.

Vorzugsweise ist die Längsachse des Probensammelrohres in einem Winkel von annähernd 45° zur Horizontalen angeordnet, wodurch ein annähernd gleichmäßiges Durchströmen des Probensammelrohres erreicht wird.

Hinter dem Auslaßventil kann weiterhin ein Gasmengenmesser angeordnet sein.

Es ist auch möglich, die Vorrichtung an den Ausatemschenkel einer Beatmungsvorrichtung mit offenem System anzuschließen.

Die Erfindung soll nachstehend an einem Ausführungsbeispiel näher erläutert werden. In der zugehörigen Zeichnung zeigen:
- Fig. 1: die Vorderansicht der Vorrichtung zum Sammeln von ausgeatmetem Atemkondensat im Schnitt,

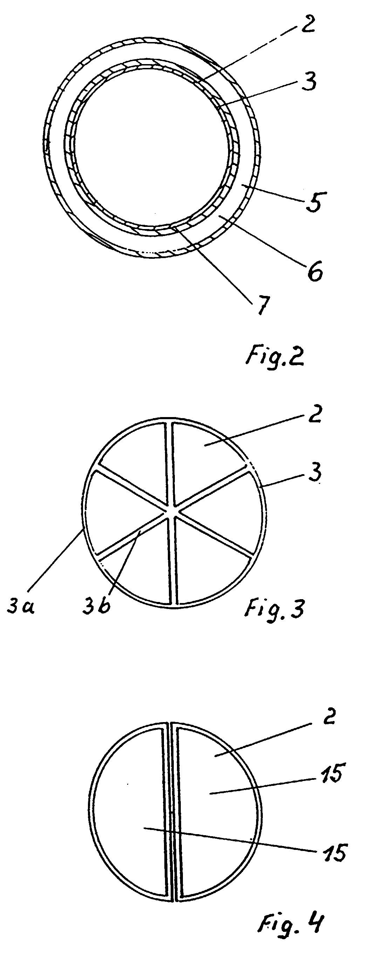

- Fig. 2: den Schnitt A-B nach Fig. 1,
- Fig. 3: den Querschnitt eines Probensammelrohres mit Innenwänden,
- Fig. 4: den Querschnitt eines mehrteiligen Probensammelrohres,
- Fig. 5: die Vorderansicht der Vorrichtung in einer weiteren Ausbildung,
- Fig. 6: Diagramme des Atemkondensates.

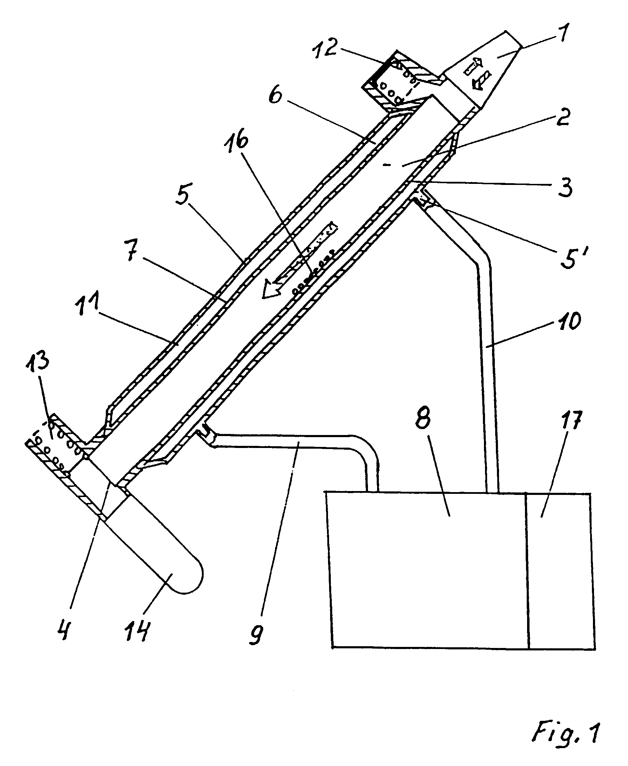

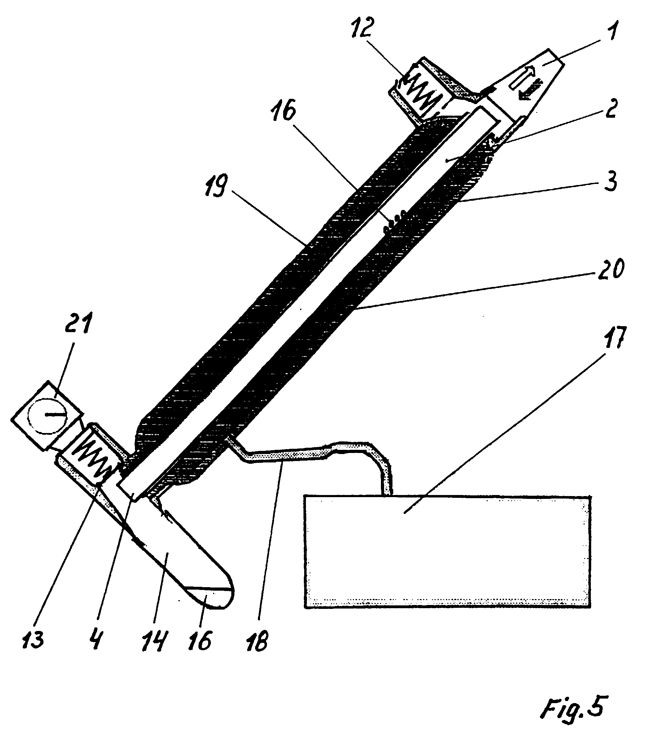

Zum Sammeln von ausgeatmetem Atemkondensat 16 einer zu untersuchenden Person, wie eines Patienten oder eines Probanden, atmet dieser über ein Mundstück 1 von oben in ein schräg liegendes Probensammelrohr 2 aus. Das Probensammelrohr 2 kann Teil der Vorrichtung gemäß Fig. 1 oder Fig. 5 bzw. weiterer Ausbildungen sein. Die Ausatemluft wird im Probensammelrohr 2 auf eine Temperatur von unter 0 °C bis etwa -5 °C abgekühlt. Dabei kondensieren alle flüssigen und löslichen Bestandteile. Sie frieren an der Innenwand 3 an, da diese selbst auf eine Temperatur bis zu -25 °C abgekühlt wird.

Verbleibende Reste des Atemkondensates 16 können in einem dem unteren Ende 4 des Probensammelrohres 2 nachgeordneten Sammelgefäß 14 aufgefangen werden. Grundsätzlich schlägt sich das Atemkondensat 16 jedoch an der Innenwand 3 des Probensammelrohres 2 nieder.

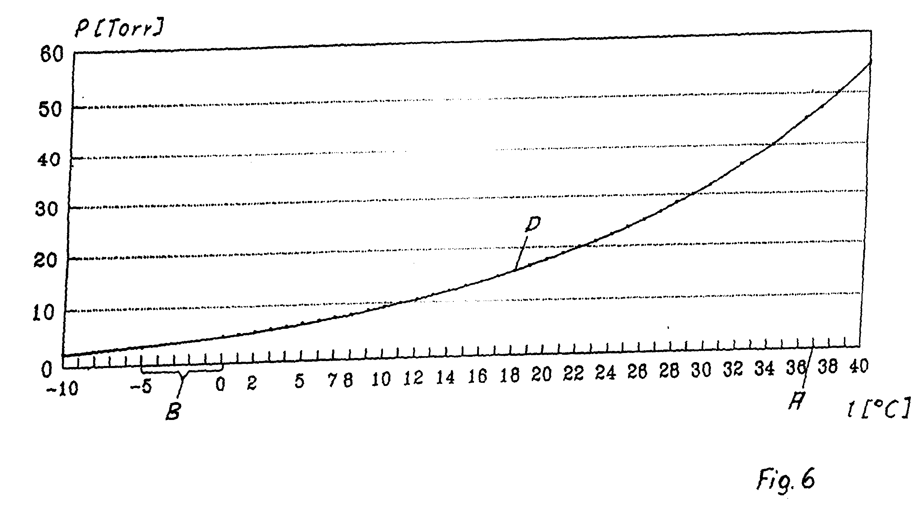

In Fig. 6 ist in einem Diagramm der Wasserdampf-Sättigungsdruck des Atemkondensates 16 in Abhängigkeit von der Temperatur gezeigt. Dabei ist auf der Abszissenachse die Temperatur t in °C und auf der Ordinatenachse der Druck p in Torr dargestellt. Die Wasserdampf-Sättigungskurve C zeigt, wie mit sinkender Temperatur von der Temperatur A bei der Ausatmung bis zur Temperatur B im Bereich des unteren Endes 4 des Probensammelrohres 2 der Wasserdampf-Sättigungsdruck des Atemkondensates 16 sinkt und damit der Niederschlag an der Innenwand 3 des Probensammelrohres 2 gewährleistet wird.

An dieser Innenwand 3 werden die für eine Untersuchung maximal erforderlichen 5 ml Primärkondensat gesammelt, welche bei einem Atemvolumen von 100 - 200 l entstehen. Hierzu sind etwa bis zu 15 min Ruheatmung der zu untersuchenden Person erforderlich.

Das Probensammelrohr 2 und das Sammelgefäß 3 werden nach Abschluß des Sammelvorganges verschlossen und bis zur Analyse der Probe in einem Tiefkühlschrank aufbewahrt.

Eine weitere Möglichkeit besteht darin, das ausgeatmete Atemkondensat 16 in einem mehrteiligen Probensammelrohr 2 (Fig. 4) zu sammeln. Hierdurch entstehen unter gleichen Bedingungen mehrere Proben. Diese können dann zu unterschiedlichen Zeiten analysiert werden. In einem gut verschlossenen Probensammelrohr 2 ist es möglich, die Probe bis zu drei Jahren aufzubewahren.

Die gesammelte Probe kann in an sich bekannter Weise zur Analyse mit Methoden der Gefriertrocknung, chemischen Fällungen oder Fraktionierungen weiter eingeengt bzw. in unterschiedliche Stoffgruppen aufgespalten werden.

Mit der Methode des Radioimmunoassay (RIA), des Enzymimmunoassay (EIA) und der Gaschromatographie können aus der Probe Mediatoren und Stoffwechselprodukte (Leukotriene, Prostaglandine, Tumormarker, Antikörper, andere Gewebshormone, Kohlenwasserstoffe) bestimmt werden.

Die gewonnenen Proben können ferner mit allen laborüblichen Analysemethoden, wie alle Arten der Chromatographie, HPLC, Gaschromatographie, Massenspektrometrie, Elektrophoresen, Fluoreszenz- und Laserspektroskopie u. ä., weiter analysiert werden.

Durch die Untersuchungen sind Messungen von Stoffwechselprodukten aus Zellen der Atemwege und ausgeatmeter Stoffwechselprodukte anderer Organe, die über die Blutbahn in die Lunge gelangen, weiter die Analyse ausgeatmeter körperfremder Stoffe, die vorher über die Atemwege oder über die Nahrung und die Haut in den Organismus gelangt sind, möglich.

Sie dienen, wie alle auf ähnlichen Methoden basierende Labormethoden, dazu, Entzündungsreaktionen, Krebswachstum, Allergien, degenerative Prozesse und auf den Organismus wirkende Umwelteinflüsse zu analysieren. Dabei sind punktuelle Bestimmungen und Verlaufsbeobachtungen mit Mehrfachbestimmungen zu trennen.

Das erfindungsgemäße Verfahren zum Sammeln von ausgeatmetem Atemkondensat ist für jeden Patienten zumutbar und kann von jedem Patienten, der ohne fremde Hilfe atmen kann, beliebig oft durchgeführt werden. Der Zeitaufwand von bis zu 15 min erscheint auch für Routineuntersuchungen und Screeningtests möglich.

Durch die geforderte Ruheatmung des Patienten handelt es sich um ein Verfahren, bei dem die gewünschte Probe nicht durch die Sammelmethode verändert wird, wie es z. B. bei einer forcierten Atmung der Fall wäre.

Mit dem Verfahren ist es auch möglich, Atemkondensat von künstlich beatmeten Patienten bei Verfahren der Beatmung im offenen System zu sammeln. Hierbei wird das Probensammelrohr 2 am Ausatemschenkel angeschlossen.

Entsprechend der in Fig. 1 und Fig. 5 dargestellten Vorrichtung ist das Probensammelrohr 2 in einem sich entlang seiner Längsachse erstreckenden Kühlelement herausnehmbar angeordnet, wobei das Kühlelement mit einem Elektrik- und Steuerteil 17 verbunden ist.

In Fig. 1 ist die Ausführungsform einer Vorrichtung zur Durchführung des Verfahrens dargestellt. Das Probensammelrohr 2 ist wie dargelegt entnehmbar in der Vorrichtung angeordnet. Es besteht vorzugsweise aus einem Kunststoff. Seine Innenwand 3 muß dabei so ausgebildet sein, daß das Atemkondensat 16 gut anfriert und nach Erwärmung sich vollständig löst. Dabei dürfen keine chemischen Reaktionen zwischen dem Atemkondensat 16 und dem Material des Probensammelrohres 2, auch bei wiederholter Verwendung desselben, eintreten. Ein besonders geeignetes Material hierfür ist Polytetrafluoräthylen, auch unter dem warenzeichenrechtlich geschützten Handelsnamen Teflon bekannt. Das Probensammelrohr 2 weist eine Länge von 10 bis 100 cm sowie einen Innendurchmesser von etwa 2 cm auf. Damit ist bei einer entsprechenden Kühlung des Atemkondensates 16 ein vollständiger Niederschlag und Anfrieren desselben an der Innenwand 3 des Probensammelrohres 2 gewährleistet.

Die Vorrichtung weist als Kühlelement ein zylindrisches Kühlmantelrohr 5 auf, das an seinem oberen und unteren Ende mit Stutzen 5' versehen ist. Das Kühlmantelrohr 5 ist über an den Stutzen 5' angeordnete Leitungen 9; 10 mit einem Kühlaggregat 8 mit einer Umwälzpumpe verbunden, und sein Innenraum 6 wird während des Betriebes ständig von einer Kühlflüssigkeit 11 durchströmt. Diese tritt vorzugsweise am unteren Ende des Kühlmantelrohres 5 ein und an dessen oberen Ende aus, so daß das Atemkondensat 16 im Probensammelrohr 2 im Gegenstromprinzip gekühlt wird. Die Kühlflüssigkeit 11 besteht vorzugsweise aus destilliertem Wasser mit einem Kühlmittelzusatz, wodurch eine Temperatur von bis zu -25 °C erreichbar ist. An der Innenseite 7 des Kühlmantelrohres liegt das Probensammelrohr 2 an und wird von diesem gekühlt. Die Temperatur an der Innenwand 3 des Probensammelrohres 2 entspricht dabei etwa der der Kühlflüssigkeit 11, während die Temperatur der seinen Innenraum durchströmenden Ausatemluft unter 0 °C bis -5 °C beträgt, wobei sie am hinteren Ende 4 desselben am niedrigsten liegt. Am vorderen Ende der Vorrichtung ist ein auswechselbares Mundstück 1 angeordnet. Am hinteren Ende der Vorrichtung befindet sich ein abnehmbares Sammelgefaß 14, in welches etwaige Restmengen des Atemkondensates 16 aus dem Probensammelrohr 2 tropfen können. Das Sammelgefäß 14 kann ebenfalls aus einem Kunststoff, wie Polytetrafluoräthylen, bestehen. Der Elektrik- und Steuerteil 17 ist an das Kühlaggregat 8 angeschlossen.

Zur Erreichung der angestrebten Ruheatmung ist an dem vorderen Ende der Vorrichtung ein Einlaßventil 12 und an seinem hinteren Ende ein Auslaßventil 13, beide in Form eines Rückschlagventils 13, angeordnet. Während des Betriebes ist die Vorrichtung in einem Winkel von etwa 45° zur Horizontalen angeordnet, da in dieser Lage ein optimales Sammeln von Proben erreicht wird. Hierzu kann die Vorrichtung in einer nicht dargestellten Einrichtung eingebaut sein.

Bei der Probennahme atmet, wie vorstehend bereits beschrieben, die zu untersuchende Person über das Mundstück 1 in das Probenrohr 2 aus. Zur Erleichterung des Atemvorganges sowie zur Vermeidung einer Rückatmung dient das Einlaßventil 12, über welches der zu untersuchenden Person Frischluft beim Einatmen zufließt, während es beim Ausatmen geschlossen ist. Während sich das Atemkondensat 16 aus der Ausatemluft an der Innenwand 3 des Probensammelrohres 2 niederschlägt, strömt der gasförmige Teil derselben über das Auslaßventil 13 in die freie Atmosphäre. Dadurch wird der Aufbau eines Überdrucks in der Vorrichtung verhindert.

Nach Abschluß der Probennahme werden das Probensammelrohr 2 und das Sammelgefaß 14 entnommen sowie das Mundstück 1 entfernt und ungebrauchte Teile eingesetzt. Die Vorrichtung ist damit wieder einsatzbereit.

In Fig. 3 ist ein Probensammelrohr 2 gezeigt, dessen Innenwand 3 durch die innere Umfangsfläche 3a des Probensammelrohres 2 und darin angeordnete Zwischenwände 3b gebildet ist. Damit wird die Fläche zum Niederschlag des Atemkondensates 16 vergrößert, wodurch insbesondere eine Verkürzung der Baulänge erreichbar ist.

In Fig. 4 ist ein aus mehreren Teilen 15 bestehendes Probensammelrohr 2 dargestellt. Bei dieser Ausbildung werden gleichzeitig mehrere Proben gesammelt, die, wie vorstehend beschrieben, zu unterschiedlichen Zeitpunkten analysiert werden können. Hierbei ist es auch möglich, das Kühlmantelrohr 5 mit einer den Innenraum der Vorrichtung unterteilenden Zwischenwand zu versehen, so daß auch die Zwischenwände des mehrteiligen Probensammelrohres 2 auf eine ein sicheres Anfrieren des Atemkondensates 16 gewährleistende Temperatur abkühlbar sind.

In Fig. 5 ist eine weitere Ausführungsform der Vorrichtung gezeigt. Diese Vorrichtung weist als Kühlelement einen piezoelektrischen Kälteteil 19 auf, der das Probensammelrohr 2 umschließt. In dem Kälteteil 19 sind ein oder mehrere piezoelektrische Elemente zur Kühlung angeordnet. Damit ist es über deren Regelung möglich, daß der Kälteteil 19 entlang dem Probensammelrohr 2 unterschiedliche Temperaturgradienten aufweist. An der Außenseite ist der piezoelektrische Kälteteil 19 mit einer Isolierung 20 versehen. Diese kann auch an dem Kühlmantelrohr 5 der Vorrichtung nach Fig. 1 angeordnet sein.

Der Kälteteil 19 ist über ein Leitungssystem 18 mit dem Elektrik- und Steuerteil 17 verbunden. Dieses umfaßt die bei piezoelektrischer Kühlung in an sich bekannter Weise vorgesehene elektrische Zuführung und Wärmeableitung.

Bei der in Fig. 5 dargestellten Vorrichtung ist hinter dem Auslaßventil ein Gasmengenmesser 21 angeordnet. Mit diesem ist die Menge der Ausatemluft bestimmbar. Es ist damit möglich, diese in das Verhältnis zum gesammelten Atemkondensat 16 zu setzen.

Der Gasmengenmesser 21 kann auch bei der Vorrichtung entsprechend Fig. 1 vorgesehen sein.

Der weitere Aufbau dieser Vorrichtung entspricht der in Fig. 1 gezeigten Vorrichtung.

In nicht dargestellter Weise ist es auch möglich, daß das, das Probensammelrohr 2 umschließende, Kühlelement als ein Peltier-Element für Kühlung ausgebildet ist.

Mit dem erfindungsgemäßen Verfahren und den Vorrichtungen zur Durchführung desselben ist es möglich, in relativ einfacher und kostengünstiger Weise Proben von zu untersuchenden Personen zu sammeln, die dann an einer dritten Stelle analysiert werden können.

## Patentansprüche

1. Verfahren zum Sammeln von ausgeatmetem Atemkondensat für die Diagnose des Gesundheitszustandes und von Stoffwechselleistungen der Lunge und der Atemwege sowie anderer Organe und zur Analyse ausgeatmeter körperfremder Stoffe, wobei die Ausatemluft gekühlt und die nicht gasförmigen Bestandteile in einem Probengefäß auskondensiert werden, dadurch gekennzeichnet, daß die Ausatemluft ein Probensammelrohr (2) durchströmt und in diesem auf eine Minustemperatur unter 0 °C abgekühlt wird, wobei die flüssigen und lösbaren Bestandteile auskondensieren und an der, eine niedrigere Temperatur als das Atemkondensat (16) aufweisenden, Innenwand (3) des Probensammelrohres (2) anfrieren.

2. Verfahren nach Anspruch 1, dadurch gekennzeichnet, daß die Ausatemluft in dem Probensammelrohr (2) auf eine Temperatur von bis -5 °C abgekühlt wird und die Temperatur seiner Innenwand (3) zwischen -10 °C und -25 °C beträgt.

3. Verfahren nach Anspruch 2, dadurch gekennzeichnet, daß entlang dem Probensammelrohr (2) an der Kondensierstrecke der Ausatemluft unterschiedliche Temperaturgradienten eingestellt werden.

4. Verfahren nach Anspruch 1 und 3, dadurch gekennzeichnet, daß das Probensammelrohr (2) während der Auskondensierung kontinuierlich gekühlt wird.

5. Verfahren nach Anspruch 1, dadurch gekennzeichnet, daß in dem Probensammelrohr (2) in einem Zeitraum von bis zu 15 min bis zu fünfmal Primärkondensat gesammelt wird.

6. Verfahren nach Anspruch 1 bis 5, dadurch gekennzeichnet, daß dem Probensammelrohr (2) ein Sammelgefäß (4) nachgeschaltet wird, in dem sich bildendes Atemkondensat (16) aufgefangen wird.

7. Verfahren nach Anspruch 1 bis 6, dadurch gekennzeichnet, daß das Probensammelrohr (2) mit dem Atemkondensat (16) in gefrorenem Zustand sowie mögliche Reste des Atemkondensates (16) in dem Sammelgefäß (14) verschlossen in einem Tiefkühlschrank bis zu einer Analyse bzw. zur weiteren Aufbereitung für eine Analyse aufbewahrt werden.

8. Verfahren nach Anspruch 7, dadurch gekennzeichnet, daß die Aufbewahrungszeit bis zu drei Jahren beträgt.

9. Verfahren nach Anspruch 1 bis 8, dadurch gekennzeichnet, daß das Probensammelrohr (2) aus mehreren unabhängigen Teilen (15) gebildet wird, in denen unter gleichen Bedingungen zur gleichen Zeit das Atemkondensat (16) gesammelt und getrennt aufbewahrt wird.

10. Vorrichtung zur Durchführung des Verfahrens nach Anspruch 1 bis 9, dadurch gekennzeichnet, daß das Probensammelrohr (2) in einem sich entlang seiner Längsachse erstrekkenden Kühlelement herausnehmbar angeordnet ist, wobei das Kühlelement mit einem Elektrik- und Steuerteil (17) verbunden ist, und dabei an dem vorderen Ende der Vorrichtung ein Mundstück (1) sowie ein Einlaßventil (12) zur Zuführung von Frischluft in die Einatemluft und am hinteren Ende derselben ein Auslaßventil (13) für die gasförmigen Bestandteile der Ausatemluft angeordnet sind.

11. Vorrichtung nach Anspruch 10, dadurch gekennzeichnet, daß das Probensammelrohr (2) aus einem biologisch inerten, kälte- und gegen Chemikalien beständigen Kunststoff besteht, an dessen Oberfläche ein gutes Anfrieren und nach dem Auftauvorgang ein vollständiges Lösen des Atemkondensates (16) möglich ist.

12. Vorrichtung nach Anspruch 11, dadurch gekennzeichnet, daß der Kunststoff ein Polytetrafluoräthylen ist.

13. Vorrichtung nach Anspruch 9 bis 12, dadurch gekennzeichnet, daß die Länge des Probensammelrohres (2) zwischen 10 und 100 cm und sein Innendurchmesser bis zu 2 cm beträgt.

14. Vorrichtung nach Anspruch 10, dadurch gekennzeichnet, daß das Kühlelement als ein Kühlmantelrohr (5) ausgebildet ist, in dessen vorderem und hinterem Bereich jeweils eine Leitung (9; 10) angeordnet ist, über die es mit einem Kühlaggregat (8) verbunden und von einer, das Probensammelrohr (2) umfließenden, Kühlflüssigkeit (11) durchströmt ist.

15. Vorrichtung nach Anspruch 10 und 14, dadurch gekennzeichnet, daß der Elektrik- und Steuerteil (17) an das Kühlaggregat (8) angeschlossen ist.

16. Vorrichtung nach Anspruch 14 und 15, dadurch gekennzeichnet, daß die Ausatemluft im Probensammelrohr (2) und die Kühlflüssigkeit (11) im Innenraum (6) des Kühlmantelrohres (5) im Gegenstromprinzip zueinander fließen.

17. Vorrichtung nach Anspruch 14 bis 16, dadurch gekennzeichnet, daß die Kühlflüssigkeit (11) aus destilliertem Wasser mit einem Zusatz von Frostschutzmitteln gebildet ist, wobei der Gefrierpunkt derselben unter -25 °C liegt.

18. Vorrichtung nach Anspruch 10, dadurch gekennzeichnet, daß das, das Probensammelrohr (2) umschließende, Kühlelement als ein piezoelektrischer Kälteteil (19) ausgebildet ist, in dem ein oder mehrere piezoelektrische Elemente zur Kühlung angeordnet sind.

19. Vorrichtung nach Anspruch 18, dadurch gekennzeichnet, daß die piezoelektrischen Elemente zur Kühlung unterschiedlich regelbar sind und über diese der piezoelektrische Kälteteil (19) entlang dem Probensammelrohr (2) unterschiedliche Temperaturgradienten aufweist.

20. Vorrichtung nach Anspruch 10, dadurch gekennzeichnet, daß das, das Probensammelrohr (2) umschließende, Kühlelement als ein Peltier-Element für Kühlung ausgebildet ist.

21. Vorrichtung nach Anspruch 10, dadurch gekennzeichnet, daß am hinteren Ende der Vorrichtung unterhalb des hinteren Endes(4) des Probensammelrohres (2) ein abnehmbares Sammelgefäß (14) zur Aufnahme von möglichen Resten des Atemkondensates (16) angeordnet ist, wobei dieses aus dem gleichen Material wie das Probensammelrohr (2) besteht.

22. Vorrichtung nach Anspruch 10, dadurch gekennzeichnet, daß das Einlaßventil (12) und das Auslaßventil (13) als Rückschlagventile ausgebildet sind.

23. Vorrichtung nach Anspruch 10 bis 13, dadurch gekennzeichnet, daß die Innenwand (3) des Probensammelrohres (2) als die innere Umfangsfläche (3a) desselben sowie in diesem angeordnete Zwischenwände (3b) ausgebildet sind.

24. Vorrichtung nach Anspruch 10 bis 13, dadurch gekennzeichnet, daß das Probensammelrohr (2) aus mehreren unabhängigen Teilen (15) ausgebildet ist, welche gemeinsam von dem Kühlmantelrohr (5) umschlossen sind.

25. Vorrichtung nach einem oder mehreren der Ansprüche 10 bis 24, dadurch gekennzeichnet, daß die Längsachse des Probensammelrohres (2) in einem Winkel von annähernd 45° zur Horizontalen angeordnet ist.

26. Vorrichtung nach einem oder mehreren der Ansprüche 10 bis 25, dadurch gekennzeichnet, daß hinter dem Auslaßventil (13) ein Gasmengenmesser (21) angeordnet ist.

27. Vorrichtung nach einem oder mehreren der Ansprüche 10 bis 26, dadurch gekennzeichnet, daß diese an dem Ausatemschenkel einer Beatmungsvorrichtung mit offenem System angeschlossen ist.

## Claims

1. Process for collecting expired breath condensate for the diagnosis of the physical condition and metabolic performance of the lung and the respiratory system as well as other organs and for analysing expired exagenous substances and while doing so the expired air is cooled and the non-gaseous components condense in a sample vessel characterized by the fact that the expired air flows through a sample tube (2) where it is cooled down to a minus temperature below 0°C whereby the liquid and soluble components condense and freeze on the inside wall (3) of the sample tube (2) which has a lower temperature than the breath condensate (16).

2. Process according to claim 1 characterized by the fact that the expired air in the sample tube (2) is cooled down to a temperature of up to -5°C and the temperature of its inside wall (3) is between -10°C and -25°C.

3. Process according to claim 2 characterized by the fact that different temperature gradients are adjusted at the condensating distance of the expired air along the sample tube (2).

4. Process according to claim 1 and 3 characterized by the fact that the sample tube (2) is continuously cooled during the condensation.

5. Process according to claim 1 characterized by the fact that primary condensate is collected in the sample tube (2) up to 5 times within a period of 15 min.

6. Process according to claim 1 to 5 characterized by the fact that a collecting vessel (4) in which the breath condensate (16) is collected, is connected at the outlet side of the sample tube (2).

7. Process according to claim 1 to 6 characterized by the fact that the sample tube (2) with the frozen breath condensate (16) as well as possible rests of the breath condensate (16) in a closed collecting vessel (14) are stored in a deep-freeze cabinet until they are analysed or further prepared for an analysis.

8. Process according to claim 7 characterized by the fact that the storage time can be up to 3 years.

9. Process according to claim 1 to 8 characterized by the fact that the sample tube (2) consists of several individual parts (15) in which the breath condensate (16) is collected and stored separately under the same conditions and at the same time.

10. Device for performing the process according to claim 1 to 9 characterized by the fact that the removable sample tube (2) is arranged in a cooling element which reaches along its longitudinal axis and the cooling element is connected with an electric unit and a control unit (17) and a mouthpiece (1) as well as an inlet valve (12) for suppling fresh air to the inspiratory air are arranged at the front end of the device and an outlet valve (13) for the gaseous components of the expiratory air is arranged at the rear end of the device.

11. Device according to claim 10 characterized by the fact that the sample tube (2) consists of plastic material which is biologically inerted, cold-resistant and resistant against chemicals and which allows good freezing on its surface and complete removing of the breath condensate (16) after defrosting.

12. Device according to claim 11 characterized by the fact that the plastic material is polytetrafluorethylene.

13. Device according to claim 9 to 12 characterized by the fact that the length of the sample tube (2) is between 10 and 100 cm and its inside diameter is up to 2 cm.

14. Device according to claim 10 characterized by the fact that the cooling element is designed as a cooling jacket tube (5) which has one line each (9; 10) at its front and rear area so that it is connected with the refrigerator (8) and a cooling fluid (11) flows around the sample tube (2).

15. Device according to claim 10 and 14 characterized by the fact that electric and control part (17) are connected to the refrigerator (8).

16. Device according to claim 14 and 15 characterized by the fact that the expired air in the sample tube (2) and the cooling fluid (11) in the inside space (6) of the cooling jacket tube (5) flow to each other according to the counter-flow principle.

17. Device according to claim 14 to 16 characterized by the fact that the cooling fluid (11) consists of distilled water and an addition of anti-freezing agents the freezing point of which is below -25°C.

18. Device according to claim 10 characterized by the fact that the cooling element which surrounds the sample tube (2) is designed as a piezoelectric cooling part (19) in which one or several piezoelectric elements for cooling are arranged.

19. Device according to claim 18 characterized by the fact that the piezoelectric elements for cooling can be controlled individually so that the piezoelectric cooling part (19) shows different temperature gradients along the sample tube (2).

20. Device according to claim 10 characterized by the fact that the cooling element which surrounds the sample tube (2) is designed as a Peltier element for cooling.

21. Device according to claim 10 characterized by the fact that a removable collecting vessel (14) for collecting possible rests of the breath condensate (16) is connected to the rear end of the device below the rear end (4) of the sample tube (2) whereby this collecting vessel is made of the same material like the sample tube (2).

22. Device according to claim 10 characterized by the fact that the inlet valve (12) and the outlet valve (13) are designed as check valves.

23. Device according to claim 10 to 13 characterized by the fact that the inside wall (3) of the sample tube (2) forms its inner peripheral area (3a) and there are partition walls (3b) in the sample tube.

24. Device according to claim 10 to 13 characterized by the fact that the sample tube (2) consists of several individual parts (15) which are surrounded by one cooling jacket tube (5).

25. Device according to one or several of the claims 10 to 24 characterized by the fact that the longitudinal axis of the sample tube (2) is arranged in an angle of almost 45°C to the horizontal line.

26. Device according to one or several of the claims 10 to 25 characterized by the fact that a gas meter (21) is installed behind outlet valve.

27. Device according to one or several of the claims 10 to 26 characterized by the fact that it is connected to expiration elbow piece of a ventilator with open system.

## Revendications

1. Un procédé permettant de recueillir un condensat de souffle expiré et servant de base pour le diagnostic de l'état de santé et des rendements du métabolisme du poumon et des voies aériennes ainsi que d'autres organes. De plus, il rend possible l'analyse de substances expirées étrangères au corps. A cet effet, l'air expiré est réfrigéré et les ingrédients non gazeux sont condensés dans un récipient d'échantillon.
L'air expiré passe par un tube collecteur d'échantillon et y est réfrigéré à une température en dessous de 0 °C. Les substances liquides et solubles condensent et prennent en gelant sur la paroi intérieure (3) du tube collecteur (2) dont la température est inférieure à celle du condensat de souffle (16).

2. Procédé conformément à réclamation 1 : l'air expiré est réfrigéré jusqu'à -5 °C dans le tube collecteur d'échantillon (2) dont la température de la paroi intérieure (3) est de -10°C à - 25°C.

3. Procédé conformément à réclamation 2 : il existe des différents gradients de température qui sont mis au point au long du tube collecteur d'échantillon (2), au chemin de condensation de l'air expiré.

4. Procédé conformément aux réclamations 1 et 3 : le tube collecteur d'échantillon (2) est réfrigéré continuellement durant la condensation.

5. Procédé conformément à réclamation 1 : dans le tube collecteur d'échantillon (2) est recueilli jusqu'à 5 fois du condensat primaire dans un délai de jusqu'à 15 minutes.

6. Procédé conformément aux réclamations 1 à 5 : au tube collecteur d'échantillon (2) est raccordé un récipient (4) permettant de recueillir le condensat de souffle.

7. Procédé conformément aux réclamations 1 à 6 : le tube collecteur d'échantillon (2) comprenant le condensat de souffle (16) congelé ainsi que les restes éventuels du condensat de souffle sont renfermés dans le récipient collecteur (14) et conservés dans un congélateur jusqu'une analyse ou une préparation pour une analyse sera effectuée.

8. Procédé conformément à réclamation 7 : la durée de conservation s'élève à jusqu'à trois ans.

9. Procédé conformément aux réclamations 1 à 8 : le tube collecteur d'échantillon (2) consiste en plusieurs éléments indépendants (15) dans lesquels le condensat de souffle (16) est conservé dans des pareilles conditions au même temps en forme recueillie et séparée.

10. Dispositif et exécution du procédé conformément aux réclamations 1 à 9 : le tube collecteur d'échantillon se trouve dans un élément réfrigérant qui s'étend au long de l'axe longitudinal. Le tube collecteur d'échantillon peut être enlevé. L'élément réfrigérant est raccordé à une unité électrique de commande (17). Au bout antérieur du dispositif se trouve un embout buccal (1) ) et une soupape d'admission (12) permettant à l'air fres de se mêler avec l'air inspiré. Au bout arrière une soupape de sortie (13) permet de laisser échapper les substances gazeuses de l'air expiré.

11. Dispositif conformément à réclamation 10 : le tube collecteur d'échantillon (2) est composé d'une matière plastique biologiquement inerte résistant contre le froid et les substances chimiques. La surface de cette matière permet au condensat de souffle (16) d'y congeler et de s'y détacher complètement après la décongélation.

12. Dispositif conformément à réclamation 11 : la matière plastique est un polytétrafluoréthlène.

13. Dispositif conformément à réclamation 9 à 12 : la longueur du tube collecteur d'échantillon (2) est de 10 à 100 cm et son diamètre intérieur est de jusqu'à 2 cm.

14. Dispositif conformément à réclamation 10 : l'élément réfrigérant a la fonction d'une chemise réfrigérante dans laquelle se trouve au début et au bout un câble (9; 10) la raccordant à un groupe frigorifique (8). Un liquide frigorifique (11) passe à travers l'élément réfrigérant et contourne le tube collecteur d'échantillon (2).

15. Dispositif conformément aux réclamations 10 et 14 : l'unité électrique de commande (17) est raccordée au groupe frigorifique (8).

16. Dispositif conformément aux réclamations 14 et 15 : l'air expiré et le liquide frigorifique (11) se réunissent dans le tube collecteur réfrigérant (2), à l'intérieur de la chemise réfrigérante (5) selon le principe de contre-courant.

17. Dispositif conformément aux réclamations 14 à 16 : le liquide frigorifique (11) consiste en eau distillée avec une addition de produits antigel dont le point de congélation est inférieur à -25 °C.

18. Dispositif conformément à réclamation 10 : l'élément réfrigérant renfermant le tube collecteur d'échantillon (2) a la fonction d'une unité réfrigérante piézoélectrique (19) dans laquelle se trouvent un ou plusieurs éléments piézoélectriques pour refroidir.

19. Dispositif conformément à réclamation 18 : les éléments piézoélectriques pour refroidir sont réglables et l'unité réfrigérante piézoélectrique (19) fait preuve de différents gradients de température au long du tube collecteur d'échantillon (2).

20. Dispositif conformément à réclamation 10 : l'élément réfrigérant enrobant le tube collecteur d'échantillon (2) est caractérisé comme élément Peltier pour refroidir.

21. Dispositif conformément à réclamation 10 : au bout arrière du dispositif en dessous du bout arrière (4) du tube collecteur d'échantillon (2) se trouve un récipient collecteur (14) enlevable permettant de recueillir les restes eventuels du condensat de souffle (16). Ce récipient est composé du même matériel que le tube collecteur d'échantillon (2).

22. Dispositif conformément à réclamation 10 : la soupape d'admission (12) et la soupape de sortie (13) sont des soupapes de non-retour

23. Dispositif conformément aux réclamations 10 à 13 : la paroi intérieure (3) du tube collecteur d'échantillon (2) est l'aréal circonférentiel intérieur (3a) de celui-ci et dans le tube collecteur existent des parois de séparation (3b).

24. Dispositif conformément aux réclamations 10 à 13 : le tube collecteur d'échantillon (2) consiste en plusieurs unités indépendantes (15) qui sont toutes enrobées par la chemise réfrigérante (5).

25. Dispositif conformément à une ou plusieurs réclamations 10 à 24 : l'axe longitudinal du tube collecteur d'échantillon (2) se trouve sous un angle d'approximativement 45° par rapport à l'horizontale.

26. Dispositif conformément à une ou plusieurs réclamations 10 à 25 : derrière la soupape de sortie (13) se trouve un compteur à gaz (21).

27. Dispositif conformément à une ou plusieurs réclamations 10 à 26 : le dispositif est raccordé au côté expiratoire d'un dispositif ventilatoire avec un système ouvert.
